(19) Europäisches Patentamt
European Patent Office
Office européen des brevets

(11) Publication number: **0 110 690**
**B1**

(12) # EUROPEAN PATENT SPECIFICATION

(45) Date of publication of patent specification: **26.09.90**

(51) Int. Cl.[5]: **C 07 D 213/61,** C 07 D 213/26

(21) Application number: **83307212.7**

(22) Date of filing: **25.11.83**

(54) **Preparation of (trifluoromethyl)pyridines.**

(30) Priority: **26.11.82 US 444773**
**04.08.83 US 520399**
**09.05.83 US 492983**

(43) Date of publication of application:
**13.06.84 Bulletin 84/24**

(45) Publication of the grant of the patent:
**26.09.90 Bulletin 90/39**

(84) Designated Contracting States:
**BE CH DE FR GB IT LI NL SE**

(56) References cited:
**EP-A-0 063 872**
**WO-A-79/00094**
**DE-A-2 812 607**
**US-A-4 266 064**

**The file contains technical information submitted after the application was filed and not included in this specification**

(73) Proprietor: **THE DOW CHEMICAL COMPANY**
**2030 Dow Center Abbott Road P.O. Box 1967**
**Midland Michigan 48640-1967 (US)**

(72) Inventor: **Fung, Alexander P.**
**14 Donegal Court**
**No. 6 Pleasant Hill, CA (US)**
Inventor: **Wilson, Charles A.**
**P.O. Box 1355, Marine Boulevard**
**Pittsburg, CA (US)**
Inventor: **Fujioka, George S.**
**1874 Meadow Lane**
**Walnut Creek, CA (US)**
Inventor: **Werner, John A.**
**2201 Sycamore Drive**
**No. 44 Antioch, CA (US)**

(74) Representative: **Allard, Susan Joyce et al**
**BOULT, WADE & TENNANT, 27 Furnival Street**
**London EC4A 1PQ (GB)**

**EP 0 110 690 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The present invention relates to a method of preparing (trifluoromethyl)pyridine compounds by reacting (trichloromethyl)pyridine compounds with hydrogen fluoride in the presence of a catalytic amount of a metal halide or metal halide/phosphorus halide catalyst under liquid phase conditions.

US—A—4266064 discloses the fluorination of (trichloromethyl)pyridine compounds by the vapour phase fluorination of a chloro-β-trifluoromethyl-pyridine with hydrogen fluoride in the presence of a catalyst of a fluoride of chromium, iron, nickel, manganese, cobalt or aluminium. The reaction requires the use of high temperatures. Such vapour phase reactions suffer from disadvantages including, for example, energy costs associated with elevating the temperature of the reactants, the decomposition of starting materials and end products associated with high temperature vapour phase reaction systems.

US—A—4184041 discloses the preparation of (trifluoromethyl)pyridine compounds by reacting a (trichloromethyl)pyridine compound with gaseous hydrogen fluoride at a temperature of from 0°—50°C. While this method may produce small quantities of (trifluoromethyl)pyridine compounds, it is an unacceptable commercial means of producing (trifluoromethyl)pyridine compounds.

It is clearly evident that a more efficient method of preparing (trifluoromethyl)pryidine compounds is desirable in order to commercially produce such compounds.

Accordingly, the present invention provides a method of preparing a trifluoromethyl substituted pyridine compound from a trichloromethyl substituted pyridine compound containing one or two trichloromethyl groups, which trichloromethyl pyridine compound may be substituted with one or more other substituents which do not affect the halogen exchange reaction, which process comprises contacting the trichloromethyl pyridine compound in the liquid phase with HF, the reaction being carried out in the absence of a diluent and in the presence of a catalytic amount of $FeCl_2$, $FeCl_3$, $NbCl_5$, $TaCl_5$, $WCl_6$, $SnCl_4$, $TiCl_4$ or mixtures thereof, $SbF_3$, $FeF_2$, $FeF_3$, AgF, KF, $CrF_2$ or mixtures thereof, optionally in combination with a phosphorus halide.

The present method provides a commercially efficient means of producing (trifluoromethyl)pyridine compounds in a liquid phase reaction system. The liquid phase condition provides a reaction where the desired (trifluoromethyl)pyridine compounds are produced in a selective manner. Additionally, the present liquid phase reaction may be run continuously by the addition of starting materials to a reaction vessel while the desired (trifluoromethyl)pyridine product, which generally has a boiling point less than the temperature at which the reaction is conducted, is collected by the condensation of (trifluoromethyl)-pyridine vapours.

In the practice of the present invention a (trichloromethyl)pyridine compound is contacted with hydrogen fluoride and a catalytic amount of one of the specified metal chlorides or metal fluorides, optionally in combination with a phosphorus halide hereinafter referred to as "catalyst", under liquid phase reaction conditions, and, optionally, under superatmospheric pressure.

(Trichloromethyl)pyridine compounds employed as the starting material are unsubstituted or substituted-(trichloromethyl)pyridine compounds containing one or two trichloromethyl groups. The trichloromethyl groups may be in α, β or γ ring positions. The pyridine ring optionally contains other substituents, besides the $CCl_3$ groups, which do not affect the halogen exchange reaction of this invention. Such substituents include, for example, Cl, Br, I or F. Preferred (trichloromethyl)pyridine compounds include mono- or dichloro-β-trichloro methyl pyridine, such as, 2,3-dichloro-5-trichloro methyl)-pyridine; 2-chloro-5-(trichloromethyl)-pyridine; and 2,6-dichloro-3-(trichloromethyl)-pyridine.

Hydrogen fluoride is employed as the source of fluorine in the present reaction. The hydrogen fluoride is introduced into the present reaction as hydrogen fluoride (anhydrous) of a hydrofluoric acid. The hydrogen fluoride is bubbled into the reaction as a gas or fed into the reaction as a liquid. Hydrogen fluoride (anhydrous) has a boiling point of 19.5°C and the liquid and gas consist of associated molecules. Hydrogen fluoride (anhydrous) is a well-known compound and commercially available, generally in cylinders and tank cars. Hydrogen fluoride is also supplied as hydrofluoric acid which is hydrogen fluoride in aqueous solution. In the practice of the present invention, hydrogen fluoride is contacted with the other reactants and preferably hydrogen fluoride (anhydrous) is employed as the hydrogen fluoride source. Hydrogen fluoride is supplied at a ratio of at least 3 moles per mole of mono-(trichloromethyl)pyridine compound and preferably an excess of this amount is employed. When bis-(trichloromethyl)pyridine compounds are employed as starting materials at least 6 moles of HF per mole of bis-(trichloromethyl)pyridine compound are required to fluorinate the 2 trichloromethyl groups while it is preferred to supply an excess of this amount.

The aforementioned metal chloride or metal fluoride catalysts are added to the present reaction in catalytic amounts, generally from 0.1 to 20 mole percent based on the amount of (trichloromethyl)pyridine compound starting material present, and preferably from about 0.5 to 10 mole percent. Preferred catalysts include $FeCl_3$ and $FeF_3$. Especially preferred catalysts are $FeCl_3$, $FeF_2$ and mixtures thereof.

Also acceptable as a catalyst is a combination of the aforesaid metal chloride or metal fluoride catalysts with a phosphorus halide. Such a combination is achieved by supplying a phosphorus halide to the reaction mixture in addition to the metal halide catalyst. A preferred phosphorus halide is $PCl_5$.

Catalysts bonded to inert supports or pre-cursor compounds which form the catalysts *in situ* are also useful in the present invention. Examples of inert supports to which the catalysts may be bonded include

graphite, alumina, silica, silica alumina, various clays and molecular sieves which are well known in the art.

The present reaction is conducted under liquid phase conditions at a temperature usually under 250°C, preferably at a temperature between 50°C and 190°C. It is especially preferred to conduct the present reaction at a temperature between 160°C and 180°C. The present halogen exchange reaction is typically conducted in the presence of agitation sufficient to maintain an essentially homogeneous mixture of the reactants. The present reaction may advantageously be carried out at superatmospheric pressure in the range of from 5—1200 psig (135.8—8375.04 kP), but it is convenient to conduct the reaction at ambient atmospheric pressure. A most preferred pressure is about 15 psig (204.75 kP).

In conducting the present reaction the order of addition of the reactants is not critical. Preferably, the (trichloromethyl)pyridine compound and the catalysts are admixed to form a reaction mixture and thereafter the hydrogen fluoride is added into this mixture, with stirring, until the reaction is completed, generally in from 1 to 100 hours. The exact time that the reaction is complete will vary on a variety of factors, such as, temperature, catalyst concentration, HF flow rate, degree of agitation and pressure. The hydrogen fluoride is fed into the reaction mixture as a liquid or, alternatively, may be bubbled or sparged into the reaction mixture as a gas.

In a preferred operation, 2,3-dichloro-5-(trichloromethyl)pyridine is mixed with a catalytic amount of $FeCl_3$ or $FeCl_2$ and then hydrogen fluoride (anhydrous) is continuously added into the reaction mixture while the temperature is increased to from 150°C to 190°C and preferably from 160°C to 180°C. The reaction is usually completed in from 1 to 48 hours. The hydrogen fluoride (anhydrous) and HCl which escapes from the reaction mixture as vapor is conveniently collected employing conventional techniques such as by condensation.

A unique aspect of the present invention is presented when the desired (trifluoromethyl)pyridine compound which is being prepared has a boiling point below the temperature at which the reaction is conducted. When this occurs, the (trifluoromethyl)pyridine product vaporizes as it is formed and is conveniently collected in a pure form, separate from any (trichloromethyl)pyridine starting materials which generally have boiling points greater than the temperature at which the reaction is conducted. This allows the reaction to be run continuously by the substantially continuous addition or feeding of (trichloromethyl)pyridine compound and hydrogen fluoride to the reaction mixture.

The following examples further illustrate the invention. No attempt has been made to balance any chemical equations described herein.

Example 1

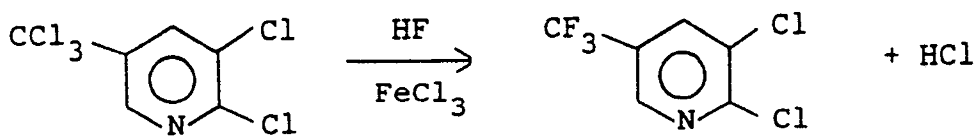


A 480 ml Teflon PFA® reaction flask fitted with a PFA reflux condenser, an HF bleed tube, a magnetic stirrer and an optical pyrometer, was charged with 220 g of 2,3-dichloro-5-(trichloromethyl)pyridine and 9.5 g of $FeCl_3$. Anhydrous HF gas was introduced into the reaction mixture below the surface of the liquid as the mixture was heated to a temperature of 175°C and maintained for a period of 14 hours. The anhydrous HF gas was bubbled into the reaction mixture throughout this 14 hour period. The reaction mixture was cooled and quenched with 100 g of ice water. The organic layer was separated, neutralized with $NaHCO_3$ and dried over $Na_2SO_4$. Analysis of the crude product employing standard gas-liquid chromatography (GLC) procedures indicated the product contained 58.1% 2,3-dichloro-3-(trifluoromethyl)pyridine, 24.4% 2,3-dichloro-5-(chlorofluoromethyl)pyridine, 7.4% 2,3-dichloro-5-(dichloromethyl)pyridine and 10% 2-fluoro-3-chloro-5-(trifluoromethyl)pyridine. The crude product was then distilled resulting in 85 g of 2,3-dichloro-5-(trifluoromethylpyridine which had a boiling point of 170—172°C.

Example 2

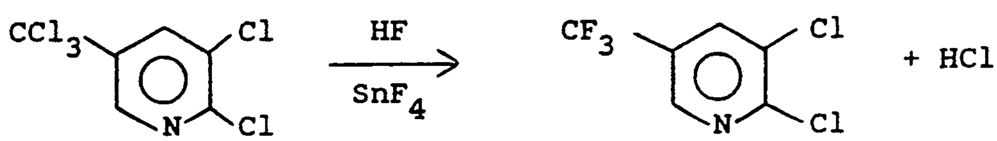


Into a 240 ml Teflon PFA® reactor was charged 70 g of 2,3-dichloro-5-(trichloromethyl)pyridine and 2.33 g of tin tetrafluoride. Anhydrous HF gas was introduced into the reaction mixture below the surface of the liquid as the mixture was heated to a temperature of 170°C over a period of 11 hours. The anhydrous HF was bubbled into the reaction period throughout this 11 hour period. Analysis of the crude product employing standard GLC procedures indicated the product contained 64% 2,3-dichloro-5-(trifluoromethyl)pyridine (includes isomers), 9.6% 2,3-dichloro-5-(chlorodifluoromethyl)pyridine, 2.2% 2,3-(dichlorofluoromethyl)pyridine and 24.2% 3-chloro-2-fluoro-5-(trifluoromethyl)pyridine.

Example 3

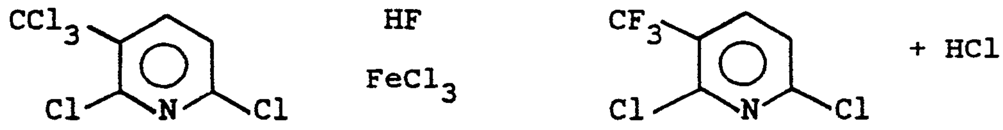


A 480 ml Teflon PFA® reaction flask fitted with a PFA reflux condenser, an HF bleed tube, a magnetic stirrer and an optical pyrometer was charged with 220 g of 2,6-dichloro-5-(trichloromethyl)pyridine and 6.71 g (5 mole %) of $FeCl_3$. Anhydrous HF gas was introduced into the reaction mixture below the surface of the liquid as the mixture was heated to a temperature of 180°C and maintained for a period of 19.5 hours. The reaction mixture was cooled and quenched with 150 g of ice water. The organic layer was separated, neutralized with $NaHCO_3$ and dried over $MgSO_4$. Analysis of the crude product employing standard GLC procedures indicated that the reaction was completed. The crude product was then distilled under reduced pressure (110°C/85 mm) resulting in 125 g of 2,6-dichloro-5-(trifluoromethyl)pyridine which corresponds to a yield of 70% of theoretical.

Example 4

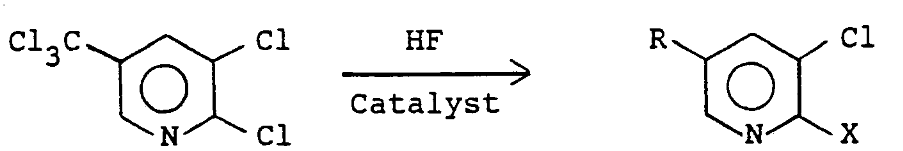


(V)

R = $CF_3$, $CF_2Cl$, $CFCl_2$ or $CCl_3$
X = Cl or F

2,3-Dichloro-5-(trichloromethyl)pyridine was reacted with HF (anhydrous) and various metal halide or metal halide/phosphorus halide catalysts under various temperature and time conditions. The products were analysed employing standard GLC procedures to determine the area percent of the reaction products (area under the curve) and conversion of starting materials. The reaction products included compounds of Formula (V) above wherein R represents $—CF_3$ (the desired product), $—CF_2Cl$, $—CFCl_2$ or $—CCl_3$ (starting material) and X represents Cl or F. Also produced is the ring fluorinated compound which is represented by Formula (V) above when R represents $CF_3$ and X represents F. The results are indicated below in Table 1.

TABLE 1

| Run # | Catalysts | Amount of Catalysts (mole %) | Reaction Time (hrs) | Reaction Temperature (°C) | Components Produced in Area Percent | | | | |
|---|---|---|---|---|---|---|---|---|---|
| | | | | | X = Cl* R = $CF_3$ | X = Cl R = $CF_2Cl$ | X = Cl R = $CFCl_2$ | X = Cl R = $CCl_3$ | X = F R = $CF_3$ |
| 1 | $SbF_3/PCl_5$ | 3.5/3.5 | 48 | ≤185 | 49 | 43 | 2 | 2 | 5 |
| 2 | $TiCl_4/PCl_5$ | 2/2 | 23 | ≤200 | 66 | 17 | 1 | 1 | 15 |
| 3 | $AgF/PCl_5$ | 2/2 | 45 | ≤180 | 51 | 37 | 8 | 1 | 2 |
| 4 | $KF/PCl_5$ | ≤100/100 | 49 | ≤185 | 3 | 12 | 71 | 11 | 3 |
| 5 | $SnCl_4$ | 4.5 | 14 | ≤180 | 43 | 15 | 2 | 1 | 39 |
| 6 | $CrF_2$ | 6.5 | 35 | 170 | 65 | 22 | 2 | 1 | 10 |
| 7 | $WCl_6$ | 1.7 | 34 | 175 | 50 | 37 | 8 | 1 | 4 |
| 8 | $TaCl_5$ | 1.2 | 22 | 165 | 59 | 32 | 5 | 0.5 | 3.5 |
| 9 | $CoCl_2$ | 4 | 22 | 175 | 1 | 12 | 67 | 20 | - |
| 10 | $NbCl_5$ | 1.6 | 15 | 175 | 43 | 25 | 12 | 1 | 19 |
| 11 | $MnF_3$ | 4 | 29 | 175 | 1 | 15 | 73 | 11 | - |
| 12 | $SnCl_4$ | - | - | 190 | o.h. 53 | 34 | 5 | - | 8 |
| 13 | $SnF_4$ | 4.5 | 11 | 170 | pot 47.5 | 43.5 | 5.7 | - | 2 |
| 14 | $FeCl_3$ | 5.2 | 18 | 170 | 66.5 | 10 | 2 | 1 | 20.5 |
| 15 | $FeCl_3$ | 5.2 | 16.5 | 190 | 48 | 47 | 1 | 0.1 | 4 |
| 16 | $FeCl_3$ | 0.5 | 15 | 170 | 46 | 42 | 1 | - | 11 |
| 17 | $FeCl_3$ | 10 | 15 | 170 | 13.3 | 60.6 | 17.6 | 2.3 | <1 |
| 18 | $FeCl_3$ | 7 | 14 | 175 | 70.1 | 8.3 | 3.4 | - | 18.2 |
| | | | | | 60 | 24 | 4 | - | 8 |

*includes the isomer where X = F and R = $CF_2Cl$

Example 5

Substantially the same procedures of Example 4 was carried out employing various catalysts under varying temperature and time conditions. The results are reported in Table 2.

TABLE 2

| Run # | Catalysts | Amount of Catalysts (mole %) | Reaction Time (hrs) | Reaction Temperature (°C) | Components Produced in Area Percent X = Cl R = $CF_3$ | X = F R = $CF_2Cl$ | X = Cl R = $CF_2Cl$ | X = Cl R = $CFCl_2$ | X = Cl R = $CCl_3$ | X = F R = $CF_3$ |
|---|---|---|---|---|---|---|---|---|---|---|
| 1 | - | - | 12 | 170 | - | - | Trace | 27 | 73 | - |
| 1a | - | - | 51.5 | ≤200 | - | 3 | 58 | 36 | 3 | - |
| 2 | $FeCl_3$ | 12.2 | 13 | 170 | 63 | 2 | 18 | <1 | - | 12 |
| | | (7.5 w%) | | | 18 | 19 | 47 | 1 | - | 3 |
| 3 | $FeCl_3$ | 12.2 | 13 | 145 | 45 | 8 | 38 | 3 | - | 9 |
| | | (7.5 w%) | | | | | | | | |
| 4 | $FeF_3$ | 7 | 12 | 170 | - | 16 | 48 | 36 | - | - |
| 5 | $PCl_5$ | 1.5 | 14.5 | 150 | 48 | 5 | 35 | 2 | - | 8 |
| 6 | $FeCl_3/PCl_5$ | 12.2/1.5 | 12.5 | 150 | 54 | 3 | 38 | - | - | 8 |
| 7 | $FeCl_3/PCl_4$ | 12.2/1.5 | 8 | 170 | 8 | 17 | 64 | 5 | - | - |
| 8 | $TiCl_4$ | 5.9 | 24 | 180 | <1 | 10 | 46 | 37 | - | - |
| 9 | $AlCl_3$ | 15 | 28 | 175 | - | - | 3 | 56 | 47 | - |
| 10 | $ZrCl_4$ | 7 | 22 | 175 | | | | | | |

Example 6

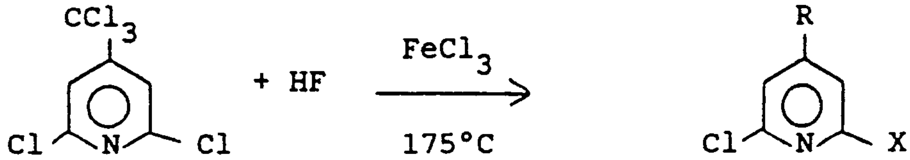


(VI)

R represents $CCl_3$, $CFCl_2$, $CF_2Cl$ or $CF_3$
X represents Cl or F.

A mixture containing 180 g of 2,6-dichloro-4-(trichloromethyl)pyridine and 12.2 g of $FeCl_3$ (11 mole%) was reacted with an excess molar amount of HF at 175°C at atmospheric pressure for 7 hours employing substantially the same procedures as set forth in Examples 1—5. Analysis of reaction product indicated the formation of the following compounds as represented in Formula (VI) above (G.C. area%):

| R = $CF_3$ | R = $CF_2Cl$ | R = $CF_2Cl$ | R = $CFCl_2$ | R = $CCl_3$ |
|---|---|---|---|---|
| X = Cl | X = F | X = Cl | X = Cl | X = Cl |
| 19.8% | 5.0% | 67.1% | 6.8% | None |

Example 7

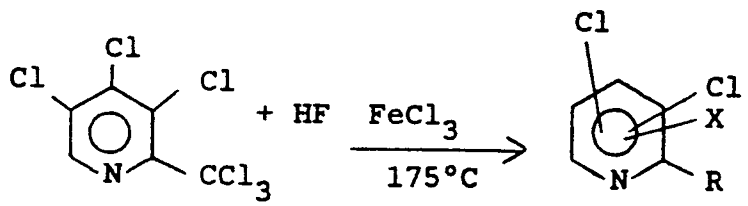


(VII)

R = $CF_3$, $CF_2Cl$, $CFCl_2$, $CCl_3$
X = Cl or F

Substantially the same procedures of Example 6 were employed except 165 g of 3,4,5-trichloro-2-(trichloromethyl)pyridine and 9.82 g of $FeCl_3$ (11 mole %) was reacted with a molar excess of HF for 26 hours at 175°C and atmospheric pressure. The product distribution in G.C. area % was as follows:

| R = $CF_3$ | R = $CF_2Cl$ | R = $CFCl_2$ | R = $CF_3$ |
|---|---|---|---|
| X = Cl | X = Cl | X = Cl | X = F |
| 30.5% | 68.5% | 0.5% | 0.5% |

Example 8

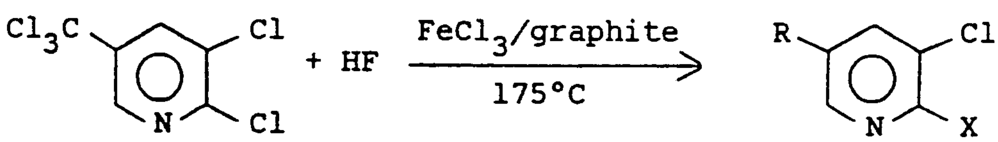


(VIII)

R = $CCl_3$, $CCl_2F$, $CClF_2$ or $CF_3$
X = Cl or F.

Substantially the same procedures of Examples 1—5 were employed except that 165 g of 2,3-dichloro-5-(trichloromethyl)pyridine and 5.76 g of $FeCl_3$ (30—40%) in graphite (equivalent to 2 mole % $FeCl_3$) was reacted with an excess molar amount of HF at 175°C at atmospheric pressure for 98 hours. Analysis of the reaction product indicated the formation of the following compounds as represented in Formula VIII above (G.C. area %) at the various times when samples were taken as listed in Table 3.

TABLE 3

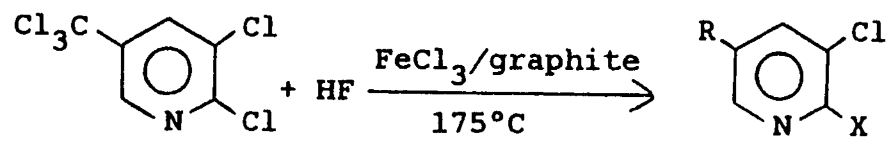


| Sample | Time (hr) | Components in Area Percent | | | | | |
|---|---|---|---|---|---|---|---|
| | | R = $CF_3$ X = F | R = $CF_3$ X = Cl | R = $CF_2Cl$ X = F | R = $CF_2Cl$ X = Cl | R = $CFCl_2$ X = Cl | R = $CCl_3$ X = Cl |
| 1 | 2 | nd* | nd | nd | 1.7 | 21.0 | 76.4 |
| 2 | 5 | nd | nd | nd | 6.7 | 57.8 | 35.5 |
| 3 | 17 | nd | 1.8 | 5.3 | 38.8 | 50.3 | 2.8 |
| 4 | 33 | 4.2 | 15.6 | 19.0 | 56.1 | 4.2 | 0.9 |
| 5 | 36 | 5.6 | 18.8 | 21.6 | 53.1 | 2.6 | 0.8 |
| 6 | 45 | 6.4 | 19.3 | 21.5 | 49.9 | 2.3 | 0.6 |
| 7 | 53 | 9.0 | 24.4 | 22.2 | 41.6 | 1.3 | 0.1 |
| 8 | 60 | 11.2 | 34.6 | 26.6 | 24.2 | 1.2 | nd |
| 9 | 74 | 13.0 | 40.1 | 21.1 | 22.9 | 1.2 | nd |
| 10 | 82 | 17.9 | 41.7 | 19.6 | 17.3 | 0.5 | nd |
| 11 | 87 | 20.4 | 49.6 | 18.2 | 11.4 | 0.3 | nd |
| 12 | 95 | 22.3 | 54.0 | 15.5 | 7.6 | 0.2 | nd |
| 13 | 98 | 23.6 | 50.7 | 14.4 | 6.6 | 0.2 | nd |

* "nd" means "not detected".

Example 9

Preparation of 2,3-dichloro-5-(trifluoromethyl)pyridine

A 71 milliliter (ml) Parr bomb (Inconel®) was charged with 16 grams (g) of 2,3-dichloro-5-(trichloromethyl)pyridine (91% purity), 7 g of anhydrous HF and 0.9 g of anhydrous $FeCl_3$. The bomb was closed and heated to a maximum of 183°C for 23 hours with rocking agitation. The maximum pressure obtained was 1150 psig (8030.26 kP). The reactor was allowed to cool to 0°C in an ice bath and the excess pressure present (about 280 psig (1930.53 kP)) was sparged into water and by analysis found to be due to HCl. A total of 15.5 g of dark brown liquid was recovered from the bomb and analyzed employing standard gas chromatography procedures. The results of the analysis corresponded to the following molecular distribution:

| | Percent (area under curve) |
|---|---|
| 2,3-dichloro-5-(trifluoromethyl)pyridine | 87.2 |
| 2,3-dichloro-5-(chlorodifluoromethyl)pyridine | 6.0 |
| 3-chloro-2-fluoro-5-(trifluoromethyl)pyridine | 2.5 |
| 2,3-dichloro-5-(dichlorofluoromethyl)pyridine | 1.2 |
| 2,3-dichloro-5-(trichloromethyl)pyridine | 0.8 |
| unidentified | 2.3 |
| | 100% |

Example 10

Preparation of 2,3-dichloro-5-(trifluoro-methyl)pyridine

A 300 ml nickel vessel, fitted with a condenser, HF feed port, sampling port, thermocouple and a pressure gauge attached to the HF feed line, was charged with 163.4 g of 2,3-dichloro-5-(trichloromethyl)pyridine (98% purity), 6.4 g of anhydrous $FeCl_3$ and sufficient anhydrous HF to maintain 15 psig (204.75 kP) pressure at a reactor temperature between 170°—180°C. Excess pressure due to HCl generation was bled out of the top of the reaction vessel through the condenser which was kept at 0°C. After 4 hours at a reaction temperature between 170°C and 180°C, almost 100% of the starting material had been converted to fluorinated methyl pyridines of the following distribution:

| | Percent (area under curve) |
|---|---|
| 2,3-dichloro-5-(chlorodifluoromethyl)pyridine | 52.7 |
| 3-chloro-2-fluoro-5-(chlorodifluoromethyl)pyridine | 33.1 |
| 2,3-dichloro-5-(trifluoromethyl)pyridine | 8.4 |
| 3-chloro-2-fluoro-5-(trifluoromethyl)pyridine | 4.9 |
| 2,3-dichloro-5-(dichlorofluoromethyl)pyridine | 0.5 |
| unidentified products | 0.5 |
| | 100% |

From the above Examples it is seen that underfluorinated materials, i.e., (chlorodifluoromethyl)pyridines and (dichlorofluoromethyl)pyridines, are present in the reaction product mixture. These underfluorinated materials are separated from the desired (trifluoromethyl)pyridine products, employing known separator techniques, and recycled into the present fluorination reaction to form the desired (trifluoromethyl)pyridine products.

Over-fluorinated materials are also formed in the present reaction, i.e., fluoro-(trifluoromethyl)pyridines or ring fluorinated pyridine compounds. These over-fluorinated materials are also readily separated from the desired (trifluoromethyl)pyridine product. When the over-fluorinated

compounds are ring fluorinated pyridine compounds wherein the fluoro attached to the pyridine ring has displaced a chloro, the ring-fluoro is displaced by chloro by reacting the over-fluorinated product with a chlorinating agent, advantageously HCl, and optionally at superatmospheric pressures. This reaction with HCl or other chlorinating agent forms the desired chloro-(trifluoromethyl)pyridines from the over-fluorinated fluoro-(trifluoromethyl)pyridine by-products. The reaction with HCl also forms chloro-(chlorodifluoromethyl)pyridines from an isomeric fluoro-(chlorodifluoromethyl)pyridine by-product. It is very desirable to reduce the amount of isomeric, i.e., fluoro-(chlorodifluoromethyl)pyridine; product because of the difficulty in separating it from the desired chloro-(trifluoromethyl)pyridine product.

In one embodiment of the present invention, the reaction product in the preparation of 2-chloro-5-(trifluoromethyl)pyridine or 2,3-dichloro-5-(trifluoromethyl)pyridine, which contains 2-fluoro-pyridines, such as, 2-fluoro-5-(trifluoromethyl)pyridine, 3-chloro-2-fluoro-5-(trifluoromethyl)pyridine, 3-chloro-2-fluoro-5-(chlorodifluoromethyl)pyridine and 2-fluoro-5-(chlorodifluoromethyl)pyridine, in addition to the desired 2-chloro- and 2,3-dibromo-5-(trifluoromethyl)pyridine and metal halide catalysts, is contacted with a chlorinating agent such as HCl at elevated temperatures to convert the 2-fluoro-pyridines to their corresponding 2-chloro analogs. The desired 2-chloro-5-(trifluoromethyl)pyridine is then readily separable from the reaction mixture by distillation since the 2-fluoro isomer, i.e. 2-fluoro-5-(chlorodifluoromethyl)pyridine, is converted to 2-chloro-5-(chlorodifluoromethyl)pyridine which has a boiling point different from the desired product. The catalyst may be added separately or may be already present in reaction product mixture as a catalyst in the preparation of 2-chloro-5-(trifluoromethyl)pyridine.

Example 11

Preparation of 2,3-Dichloro-5-(trifluoromethyl)pyridine

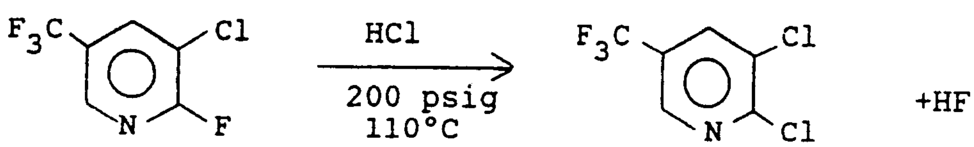


A 45 milliliter (ml) Teflon®-lined Parr bomb, which was equipped with a pressure gague, rupture disk and needle valve, was charged with 2 grams (g) of 3-chloro-2-fluoro-5-trifluoromethylpyridine and then pressurized with anhydrous HCl to 200 psig (1480.29 kP). The bomb was placed in a heated rocker and kept at 110°C for 20 hours. The maximum pressure was 220 psig (1618.19 kP). The bomb was removed from the heater and allowed to cool to room temperature, at which time it was placed in an ice bath. The bomb was vented to a caustic scrubber and 2.5 g of a light tan liquid consisting of HF and 84.3% 2,3-dichloro-5-triflurormethylpyridine (by wt.). This represents a 97.7% yield of 2,3-dichloro-5-trifluoromethylpyridine (by wt.) with 0.4% of 3-chloro-2-fluoro-5-trifluoro-methylpyridine remaining. No additional products were observed by analysis with gas chromatography.

Example 12

To a high pressure nickel reactor were added 8 grams of a mixture containing 92.6 percent by weight 3-chloro-2-fluoro-5-(trifluoromethyl)pyridine, 4.5 percent by weight 2,3-dichloro-5-(trifluoromethyl)pyridine and 2.9 percent by weight unidentified related compounds. To this mixture was added 0.7 g $FeCl_3$ (anhydrous). The reactor was then pressurized to 80 psi (551.58 kP) with anhydrous HCl at room temperature. The mixture was then heated to 180°C with rocking agitation for 20 hours. After cooling to room temperature, the excess pressure was released. The reactor was opened and the contents analyzed by standard gas-liquid chromatography (GLC) procedures. The results indicated the resulting product contained 83.5 percent by weight 2,3-dichloro-5-(trifluoromethyl)pyridine, 11.8 percent by weight 3-chloro-2-fluoro-5-(trifluoromethyl)pyridine and the balance unidentified by-product.

Example 13

A 200 ml PERFLUOROALKOXY® (PFA) reaction flask fitted with a PFA reflux condenser, an HCl bleed tube, a magnetic stirrer and an optical pyrometer was charged with 60 g of a mixture containing 54.8 g percent by weight 3-chloro-2-fluoro-5-(trifluoromethyl)pyridine and 45.2 percent by weight 2,3-dichloro-5-(trifluoromethyl)pyridine and 2.25 g of $FeCl_3$ (5 mole percent). Anhydrous HCl gas was introduced into the reaction mixture below the surface of the liquid as the mixture was heated to a temperature of 170°C. This temperature was maintained for a period of 15 hours after which the reaction mixture was quenched with 40 g of ice. The organic layer was separated, neutralized with $NaHCO_3$ and dried over $Na_2SO_4$. Analysis of the product indicated that it contained 68.5 percent by weight 2,3-dichloro-5-(trifluoromethyl)pyridine, 5 percent by weight 3-chloro-2-fluoro-5-(trifluoromethyl)pyridine and 26.4 percent 2,3-dichloro-5-(chlorodifluoromethyl)pyridine.

Example 14

A 335 ml nickel reaction vessel, equipped with a mechanical agitator, nickel reflux condenser and an HCl bleed tube, was charged with 105 g of 3-chloro-2-fluoro-5-(trifluoromethyl)pyridine and 7.5 g of anhydrous $FeCl_3$. Anhydrous HCl gas was continuously sparged into the reaction mixture at a rate of about 25 ml/min throughout the reaction. The temperature of the reaction mixture was kept between 140 and 160°C for 22.5 hours. The reaction mixture was quenched with 100 ml of ice water. The organic layer was separated, neutralized with $NaHCO_3$ and dried over $MgSO_4$. Analysis of the resulting product employing standard GLC procedures indicated 86 percent by weight was 2,3-dichloro-5-(trifluoromethyl)pyridine which was then isolated in a pure form by distillation.

Example 15

A 335 ml nickel vessel was charged with 105 g of a mixture containing 88 percent by weight 2,3-dichloro-5-(trifluoromethyl)pyridine and 12 percent by weight 3-chloro-2-fluoro-5-(chlorodifluoromethyl)-pyridine and 7 g of anhydrous $FeCl_3$. Anhydrous HCl gas was continuously sparged into the reaction mixture at a rate of about 20 ml/min as the mixture was heated to a temperature of 170°C and maintained at this temperature for 7 hours. The reaction mixture was quenched with 100 ml of ice water. The organic layer was separated, washed twice with 100 ml of water, neutralized with $NaHCO_3$ and dried over $MgSO_4$. Standard GLC analysis indicated the resulting product contained 90.1 percent by weight 2,3-dichloro-5-(trifluoromethyl)pyridine, 0.5 percent by weight 3-chloro-2-fluoro-5-(chlorodifluoromethyl)pyridine, 2.2 percent by weight 3-chloro-2-fluoro-5-(trifluoromethyl)pyridine and 7.2 percent by weight 2,3-dichloro-5-(chlorodifluoromethyl)pyridine.

Example 16

The reaction product mixture from the preparation of 2,3-dichloro-5-(trifluoromethyl)pyridine, made by fluorinating 2,3-dichloro-5-(trichloromethyl)-pyridine with HF and 5 mole % $FeCl_3$, in the liquid phase, was heated to reflux (145—170°C) at atmospheric pressure. HCl gas was bubbled into the mixture at a rate of 25—30 cc/min. The initial composition of the mixture is indicated at time "0" in Table 4 in weight percent. The final composition of the mixture is indicated in sample 9 in Table 4 after a 54 hour reaction period.

TABLE 4

Compounds Present in Mixture
in Weight Percent

| Sample | Time (hr) | 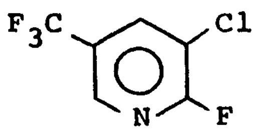 | 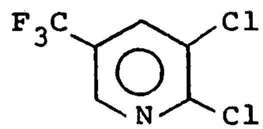 | 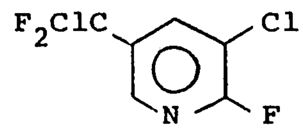 | 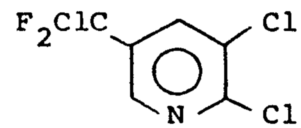 |
|---|---|---|---|---|---|
| | 0 | 81.5 | 9.9 | 4.4 | 0.8 |
| 1 | 2 | 79.0 | 11.4 | 4.2 | 0.9 |
| 2 | 4 | 75.5 | 15.5 | 4.0 | 1.1 |
| 3 | 10 | 65.4 | 25.3 | 3.3 | 1.5 |
| 4 | 14 | 53.3 | 38.0 | 1.9 | 2.2 |
| 5 | 27 | 19.2 | 70.8 | 1.1 | 4.6 |
| 6 | 32 | 14.7 | 74.3 | 0.3 | 4.7 |
| 7 | 46 | 9.0 | 78.3 | -* | 4.9 |
| 8 | 50 | 8.2 | 78.0 | -* | 5.0 |
| 9 | 54 | 7.7 | 75.8 | -* | 4.6 |

*None detected.

In another aspect of the present invention 3-chloro-2-fluoro-5-(trifluoromethyl)pyridine is prepared from 2,3-dichloro-5-(trichloromethyl)pyridine employing the procedures described herein whereby the conditions are controlled to optimize the formation of the over-fluorinated product i.e., 3-chloro-2-fluoro-5-(trifluoromethyl)pyridine. This over-fluorinated product is useful as a starting material in the preparation of 3-fluoro-5-(trifluoromethyl)pyridinyl-oxy(or thio)phenoxy propionic acid and derivatives thereof which are exceptional herbicides.

Example 17

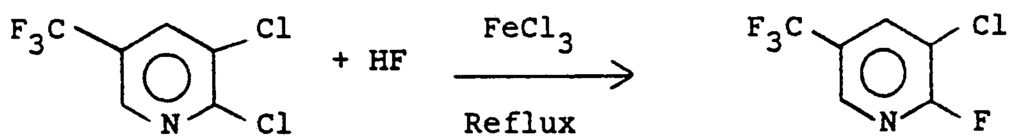


Substantially the same procedures of Example 6 were employed except that 143 g of 2,3-dichloro-5-(trifluoromethyl)pyridine (98% pure) and 4.37 g of $FeCl_3$ (5 mole %) was reacted with an excess molar amount of HF for 21 hours at 175°C and atmospheric pressure. The product distribution in G.C. area % was as follows:

| | |
|---|---|
| 2,3-dichloro-5-(trifluoromethyl)pyridine (starting material) | 67.7% |
| 3-chloro-2-fluoro-5-(trifluoromethyl)pyridine | 30.3% |

Example 18

2,3-Dichloro-5-(trifluoromethyl)pyridine

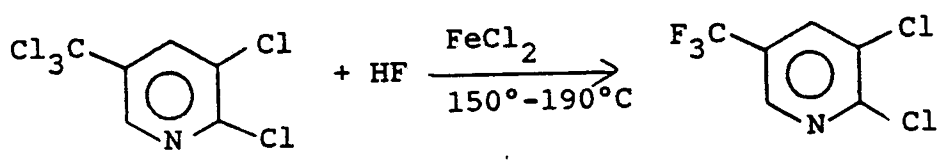


A 360 milliliter (ml) TEFLON® PFA reaction flask, fitted with a PFA reflux condenser, an HF bleed tube, a magnetic stirrer and an optical pyrometer, was charged with 180 grams (g) of 2,3-dichloro-5-(trichloromethyl)pyridine and 4.3 g (5 mole percent) of $FeCl_2$. Anhydrous HF gas was introduced into the reaction mixture (~ 4 g/hr) below the surface of the liquid as the reaction mixture was heated to a temperature between 170°C and 175°C. This temperature (170°—175°C) was maintained for a period of 70 hours with constant agitation. Standard gas-liquid chromatography (GLC) analysis of the product indicated that the reaction product contained:

| | Weight % |
|---|---|
| 2,3-dichloro-5-(trifluoromethyl)pyridine | 90.2% |
| 3-chloro-2-fluoro-5-(trifluoromethyl)pyridine | 6.1% |
| 2,3-dichloro-5-(chlorodifluoromethyl)pyridine | 3.6% |
| 3-chloro-2-fluoro-5-(chlorodifluoromethyl)pyridine | <0.1% |

Example 19

A Parr bomb, equipped with a condenser and a pressure release valve, was charged with 1,200 g of 2,3-dichloro-5-(trichloromethyl)pyridine and 28.6 g of $FeCl_2$ (5 mole %). Anhydrous HF gas was introduced into the reaction mixture (~ 3 g/hr) as the temperature was raised to 175°C. The pressure was maintained at 15

psig (204.75 kP). After 95 hours, GLC analysis of the product (after the pressure was released and the product was allowed to cool to room temperature) indicated that the reaction product contained:

| | Weight % |
|---|---|
| 2,3-dichloro-5-(trifluoromethyl)pyridine | 73.0% |
| 3-chloro-2-fluoro-5-(trifluoromethyl)pyridine | 1.7% |
| 3-chloro-2-fluoro-5-(chlorodifluoromethyl)pyridine | 0.5% |
| 2,3-dichloro-5-(chlorodifluoromethyl)pyridine | 17.5% |
| 2,3-dichloro-5-(dichlorofluoromethyl)pyridine | 2.3% |

Example 20

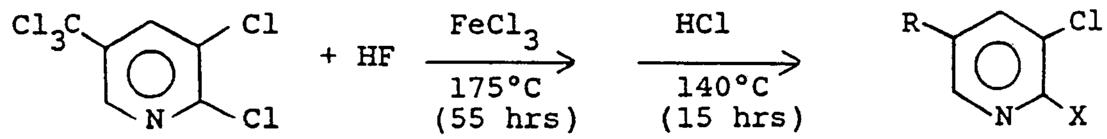


R = $CCl_3$, $CCl_2F$, $CClF_2$ or $CF_3$
X = Cl or F

Substantially the same procedures of Example 6 were repeated employing 180 g fo 2,3-dichloro-5-(trichloromethyl)pyridine and 5 mole % (5.5 g) of $FeCl_3$. The reaction was run for 55 hours. After 55 hours, the temperature of the reactants was decreased to 140°C and HCl was bubbled into the reaction mixture at a rate of 25 ml/minute for 15 hours. The product distribution in G.C. area was as follows:

| R = $CF_3$, X = Cl | R = $CF_3$, X = F | R = $CF_2Cl$, X = F | R = $CF_2Cl$, X = Cl | R = $CFCl_2$ or $CCl_3$, X = Cl |
|---|---|---|---|---|
| 79.4% | 1.8% | 0.1% | 14.2% | not detected |

**Claims**

1. A method of preparing a trifluoromethyl substituted pyridine compound from a trichloromethyl substituted pyridine compound containing one or two trichloromethyl groups, which trichloromethyl pyridine compound may be substituted with one or more other substitutents which do not affect the halogen exchange reaction, which process comprises contacting the trichloromethyl pyridine compound in the liquid phase with HF characterized in that the reaction is carried out at a temperature of below 250°C in the absence of a diluent and in the presence of a catalytic amount of $FeCl_2$, $FeCl_3$, $NbCl_5$, $TaCl_5$, $WCl_6$, $SnCl_4$, $TiCl_4$ or mixtures thereof, $SbF_3$, $FeF_2$, $FeF_3$, AgF, KF, $CrF_2$ or mixtures thereof, optionally in combination with a phosphorus halide.

2. A method as claimed in claim 1 wherein the catalyst is $FeCl_3$, $FeF_3$, $FeCl_2$ or $FeF_2$.

3. A method as claimed in claim 1 or claim 2 wherein the phosphorus halide is $PCl_5$.

4. A method as claimed in any one of claims 1 to 3 wherein the metal halide catalyst is used in an amount of from 0.5 to 10 mole percent based on the amount of (trichloromethyl)pyridine starting material present.

5. A method as claimed in any one of the preceding claims wherein the reaction is carried out at superatmospheric pressure.

6. A method as claimed in claim 5 wherein the pressure is in the range of from 5 to 1200 psig.

7. A method as claimed in any one of the preceding claims wherein the resulting ring fluorination is converted to ring chlorination by contacting the reaction mixture with a chlorinating agent.

8. A method as claimed in claim 7 wherein the chlorinating agent is HCl.

9. A method as claimed in any one of the preceding claims wherein the reaction is carried out at a temperature in the range of from 80°C to 190°C.

10. A method as claimed in claim 9 wherein the temperature is in the range of from 160°C to 180°C.

**Patentansprüche**

1. Verfahren zur Herstellung einer Trifluoromethyl-substituierten Pyridinverbindung, aud einer Trichloromethyl-substituierten Pyridinverbindung, die eine oder zwei Trichlormethyl-Gruppen enthält, wobei dei Trichlormethyl-Pyridinverbindung mit einem oder mehreren anderen Substituenten substitiuiert sein kann, welche die Halogenaustauschreaktion nicht beeinträchtigen, wobei das Verfahren die Behandlung der Trichlormethyl-Pyridinverbindung in flüssiger Phase mit HF umfaßt, dadurch gekennzeichnet, daß die Reaktion bei einer Temperatur von unterhalb 250°C ohne eine Verdünnungsmittel und in Gegenwart einer katalytischen Menge von $FeCl_2$, $FeCl_3$, $NbCl_5$, $TaCl_5$, $WCl_6$, $SnCl_4$, $TiCl_4$ oder Gemischen davon, $SbF_3$, $FeF_2$, $FeF_3$, AgF, KF, $CrF_2$ oder Gemischen davon, gegebenenfalls in Kominbation mit einem Phosphorhalogenid durchgeführt wird.

2. Verfahren nach Anspruch 1, worin der Katalysator $FeCl_3$, $FeF_3$, $FeCl_2$ oder $FeF_2$ ist.

3. Verfahren nach Anspruch 1 oder 2, worin das Phosphorhalogenid $PCl_5$ ist.

4. Verfahren nach einem der Ansprüche 1 bis 3, worin der Metallhalogenid-Katalysator in einer Menge von 0,5 bis 10 Mol-% bezogen auf die Menge an (Trichlormethyl)pyridin-Ausgangsmaterial verwendet wird.

5. Verfahren nach einem der vorhergehenden Ansprüche, worin die Reaktion oberhalb von atmosphärischem Druck durchgeführt wird.

6. Verfahren nach Anspruch 5, worin der Druck im Bereich von 5 bis 1200 psig (135,8 bis 8375,04 kP) ist.

7. Verfahren nach einem der vorhergehenden Ansprüche, worin die resultierende Ringfluorierung durch Behandlung des Reaktionsgemisches mit einem Chlorierungsmittel in eine Ringchlorierung umgewandelt wird.

8. Verfahren nach Anspruch 7, worin das Chlorierungsmittel HCl ist.

9. Verfahren nach einem der vorhergehenden Ansprüche, worin die Reaktion bei einer Temperatur im Bereich von 80°C bis 190°C durchgeführt wird.

10. Verfahren nach Anspruch 9, worin die Temperatur im Bereich von 160°C bis 180°C ist.

**Revendications**

1. Procédé de préparation d'une pyridine à substituant(s) trifluorométhyle à partir d'une pyridine à substituant(s) trichlorométhyle contenant un ou deux groupes trichlorométhyle, cette trichlorométhyl-pyridine pouvant être substituée par un ou plusieurs autres substituants qui n'affectent pas la réaction d'échange d'halogène, ce procédé comprenant la mise en contact de la trichlorométhyl-pyridine en phase liquide avec HF, caractérisé en ce que l'on effectue la réaction à une température située au-dessous de 250°C, en l'absence d'un diluant et en présence d'une quantité catalytique de $FeCl_2$, $FeCl_3$, $NbCl_5$, $TaCl_5$, $WCl_6$, $SnCl_4$, $TiCl_4$, ou de leurs mélanges, de $SbF_3$, $FeF_2$, $FeF_3$, AgF, KF, $CrF_2$ ou de leurs mélanges, éventuellement en combinaison avec un halogénure de phosphore.

2. Procédé selon la revendication 1, dans laquel le catalyseur est $FeCl_3$, $FeF_3$, $FeCl_2$ ou $FeF_2$.

3. Procédé selon la revendication 1 ou 2, dans lequel l'halogénure de phosphore est $PCl_5$.

4. Procédé selon l'une quelconque des revendications 1 à 3, dans lequel on utilise le catalyseur halogénure de métal, en une quantité allant de 0,5 à 10 pour-cent en moles par rapport à la quantité de (trichlorométhyl)pyridine de départ présente.

5. Procédé selon l'une quelconque des revendications précédentes, dans lequel on effectue la réaction à une pression supérieur à la pression atmosphérique.

6. Procédé selon la revendication 5, dans lequel la pression manométrique est comprise dans l'intervalle allant de 5 à 1200 psi (pression de 135,8 à 8375,04 kPa).

7. Procédé selon l'une quelconque des revendications précédentes, dans lequel transforme la fluoration résultante du noyau en chloration du noyau grâce à la mise en contact du mélange de réaction avec un agent de chloration.

8. Procédé selon la revendication 7, dans lequel l'agent de chloration est HCl.

9. Procédé selon l'une quelconque des revendications précédentes, dans lequel on effectue la réaction à une température comprise dans l'intervalle allant de 80° à 190°C.

10. Procédé selon la revendication 9, dans lequel la température est comprise dans l'intervalle allant de 160°C à 180°C.